# EUROPEAN PATENT APPLICATION

(11) **EP 4 647 090 A1**
(43) Date of publication of application: **12.11.2025**
(21) Application number: 24175306.0
(22) Date of filing: 10.05.2024
(51) Int. Cl.: A61L 15/32, A61F 13/01, A61L 15/44

(54) **WOUND COVER**

(71) Applicant: BioHealing s.r.o., 70800 Ostrava (CZ)
(72) Inventor: Schmiedova, Iveta, Brno (CZ); Dembickaja, Alena, Ostrava (CZ); Zahradnicek, Michal, Praha (CZ); Savic, Stefan, Pruhonice (CZ); Stastna, Elen, Praha (CZ); Gogolkova, Sona, Ostrava (CZ); Pistorova, Lucia, Vresina u Hlucina (CZ); Hyneckova, Vendula, Frycovice (CZ); Kozova, Beata, Reka (CZ)
(74) Representative: Harber IP s.r.o.

(57) **Abstract**

The invention provides a wound cover, which contains, in the following order:
- a first layer of polyester fabric,
- a first layer of petroleum jelly,
- a layer of deactivated non-viable amniotic tissue (DNAT),
- a second layer of petroleum jelly,
- a removable second layer of polyester fabric, which is configured to be removed before applying the wound cover to a wound.

The wound cover is suitable for healing acute as well as chronic wounds. It provides a suitable environment supporting the healing of the wound.

The invention further provides a method of manufacturing the wound cover.

## Description

### Field of Art

The present invention relates to amniotic membrane-based wound cover and a method of manufacture thereof.

### Background Art

Amniotic membranes are used for the manufacture of materials for reconstruction, protection and healing of injured tissues. Typically, these materials are produced by separating the amniotic membrane from the placenta, chorion, optionally removing the epithelial layer, optionally placing the amniotic membrane onto a carrier, and subsequently lyophilizing or freezing the product, or grinding the product into powder. The resulting product is therefore an amniotic membrane on a carrier, an amniotic membrane without a carrier, or a powdered product. In these products, the amniotic membrane is the source of healing substances. The procedures are described, for example, in Czech patent applications PV 2017-846, PV 2022-49, PV 2022-116.

Moist wound healing is a modern method of treating acute and chronic wounds. The wound secretes a fluid containing a number of growth factors and nutrients that contribute to successful healing. Moist healing can be supported by the use of wound covers, which maintain the naturally produced healing-promoting substances within the wound environment. However, the risk of this procedure is the undesirable proliferation of microbes that can cause inflammation in the wound.

The aim of the present invention is to create a wound cover as a medical device that could be stored under normal conditions in pharmacies and home first aid kits, and that would assist the natural processes of wound healing.

### Disclosure of the Invention

Object of the present invention is a wound cover, which contains, in the following order:
- a first layer of polyester fabric,
- a first layer of petroleum jelly,
- a layer of deactivated non-viable amniotic tissue (DNAT),
- a second layer of petroleum jelly,
- a removable second layer of polyester fabric, which is configured to be removed before applying the wound cover to a wound.

In a preferred embodiment, the wound cover contains, in the following order:
- the first layer of polyester fabric, in the amount of 3.7 to 9.8 mg/cm² of the wound cover,
- the first layer of petroleum jelly, in the amount of 6.5 to 14.7 mg/cm² of wound cover,
- the layer of deactivated non-viable amniotic tissue, in the amount of 0.2 to 4 mg/cm² of wound cover,
- the second layer of vaseline, in the amount of 5.5 to 14.7 mg/cm² of wound cover,
- the removable second layer of polyester fabric, in the amount of 3.7 to 9.8 mg/cm² of the wound cover.

The first layer of polyester fabric is the outer layer when the cover is applied to the wound, i.e. the layer facing away from the wound. Polyester fabrics are flexible and allow a good adaptation of the wound cover to the shape of the body surface at the wound site. Furthermore, they have good structural strength and therefore allow the wound cover to be removed without tearing. Another advantage is that the polyester fibers do not fuse with the newly formed tissue, so that the wound cover can be applied for a long period of time.

The second layer of polyester fabric is removable. This layer is a cover layer for packaging and transporting the cover, and this layer is removed before applying the cover to the wound. The cover is then applied to the wound with a second layer of petroleum jelly facing the wound. Due to the moisture of the wound and the properties of petroleum jelly, the cover is then kept attached to the wound, and no additional adhesive layer is needed. It is possible to cover the cover after applycation to the wound with a secondary cover, at the discretion of the physician.

A polyester fabric can be a woven or non-woven fabric made of polyester fibers. It is preferably a woven fabric. Polyester fibers can be nanofibers and/or microfibers, i.e. generally fibers with diameters from 1 nanometer to 1000 micrometers. Methods of preparing these polyester fabrics are well known in the art.

Petroleum jelly (vaselinum, vaselinum album) layers are protective layers, and thanks to their hydrophobic surface they do not allow moisture to escape from the wound, thereby contributing to the creation of a moist healing environment in the wound. Duplicating the petroleum jelly layers strengthens this effect. The second layer of petroleum jelly is the layer that adheres to the wound when the wound cover is applied.

Deactivated non-viable amniotic tissue (DNAT) is a layer that further increases the structural strength of the wound cover and is also a carrier for petroleum jelly, while not reducing the conformability of the wound cover to the shape of the wounded body area. Furthemore, DNAT provides physical and microbial protection for the wound. A test carried out according to EN ISO 22612:2005 compared a wound dressing containing only layers of polyester fabric and petroleum jelly, which resulted in the presence of such an amount of microbes that the colonies (CFU) could not even be counted, and a wound dressing according to the invention, which resulted in the presence of only an amount of microbes corresponding to 28 CFU, which represented a reduction of more than 6 logs of microbes passing through the wound dressing.

It is interesting that the amniotic tissue (DNAT) acts predominantly as a barrier against the entry of microbes into the wound and wound environment, as a layer not compromising flexibility and strength of the wound cover, and as a layer facilitating removal of the wound cover from the wound in case of need. Furthermore, DNAT is a layer which adapts well to the wound and which assists in achieving the right amount of moisture and the right environment for healing of the wound.

The wound cover according to the present invention is a disposable medical device. It has a layered structure and is sterile. If a lyophilized amniotic membrane is used, the wound cover is a semi-transparent product. Shelf life is up to 5 years at room temperature.

The wound cover according to the invention is flexible and adapts well to the shape of the wound site. The would cover is also very pleasant due to the soft two contact layers, and does not cause allergic reactions nor skin irritation. It keeps the edge of the wound flexible and protects the wound from drying out. The wound cover can be applied to the wound for up to 7 days.

The wound cover (wound dressing) according to the invention can be used to heal both acute and chronic wounds, including diabetic chronic wounds and leg ulcers. Especially in chronic wounds, communication between cells is often interrupted, cell migration is disturbed, and the formation of new tissue is inhibited, i.e. regeneration processes are inhibited. Under such conditions, it is not possible for the wound to close and for the homeostatic environment necessary for the regeneration of the damaged tissue to be created. The wound cover according to the invention provides such homeostatic moist environment, that supports cell migration and the formation of new tissue. At the same time, it prevents microbial contamination of the wound and its surroundings; and also facilitates re-epithelialization of the wound, as it acts as an extracellular matrix template and promotes the proliferation and migration of keratinocytes.

The wound cover according to the invention can be prepared by a procedure comprising the following steps:
a) preparation of amniotic membranes from amnions or from placentas containing amnions,
b) washing the amniotic membranes with physiological saline solution,
c) optional washing of amniotic membranes in an antibiotic solution,
d) lyophilization of the amniotic membrane, preferably at a temperature from +20 to - 65 °C and a pressure from 13.3 Pa to 60 Pa,
e) fragmentation of the lyophilized amniotic membrane into the required dimensions,
f) assembling the lyophilized amniotic membrane with layers of petroleum jelly and layers of polyester fabric, preferably at a temperature of 15 to 25 °C and a pressure of 101 kPa to 10 MPa, compressing the assembled layers, and placing them in sterile packaging,
g) final sterilization of the wound cover by gamma radiation with a dose of 14 to 30 kGy, preferably at a temperature from 0 to 35 °C.

In step a), the amniotic membrane is prepared from the placenta starting from the incision/rupture site. A blunt preparation is usually used. The prepared amniotic membrane is only lightly washed with physiological saline solution to remove large residues, dried, e.g. using non-woven fabric or pulp. The membrane can be used immediately for further processing, or it can be placed in a cryopack for storage before further processing, the air is sucked outffrom the cryopack and the cryopack is sealed. In the cryopack, the tissue is shock-frozen to a temperature of -40 to -80°C and can be stored for up to one year before the next processing step.

The next processing step is cleaning of the membranes, step b) and optionally also step c). Cleaning can be done manually, when the worker spreads the membrane on a table or a mat and manually removes and washes off contaminants.

Alternatively, the cleaning may be carried out by means of a washing device comprising a container with a lid, the container containing a concentrically placed perforated basket connected to a vertical central axis passing through the lid and provided with a driving means located in the lid, and the device is further provided with a fluid supply and discharge tube which opens at the bottom of the container, wherein the said tube is provided with a three-way valve outside the container, the first outlet of the three-way valve leads to the tube, the second outlet leads to a waste container, and the third outlet leads to a physiological saline reservoir. The device further contains at least one pump for pumping the physiological saline solution into the container and for pumping out the used physiological saline solution from the container after each washing cycle.

The fragmentation step e) is performed, for example, using a semi-automatic cutter. The required dimensions depend on the purpose of the wound cover, or on the target body part and on the expected maximum size of the wound.

Sterile packages commonly used for wrapping wound dressings are preferably used as sterile packaging in step f).

The wound cover according to the present invention provides a mechanical barrier for difficult-to-heal wounds of various etiologies, such as partial and full-surface wounds, bedsores, venous ulcers, diabetic ulcers, chronic vascular ulcers, surgical wounds and wounds after injuries. The wound cover according to the invention can act as protection from the external environment, prevention of liquid evaporation, and to prevent the entry of microorganisms into the wound.

All these effects work together in synergy to create an optimal homeostatic environment in the wound, which leads to faster healing of difficult-to-heal wounds. The multi-layer composition is important for effective wound healing. The gentle inner side prevents damage to the newly formed cells when repositioning or removing the device.

### Examples of carrying out the Invention

### Example 1: Production of the wound cover

Amniotic membrane was prepared from human placenta, rinsed and frozen. Subsequently, 10 amniotic membranes prepared in this manner are thawed within 30 minutes and placed in a washing device, which contains a container with a lid, wherein a perforated basket is concentrically placed in the container and connected to a vertical central axis passing through the lid and equipped with a drive located in the lid, and the device is further equipped with a tube for the supply and removal of liquids, which opens at the bottom of the container, wherein the said tube is equipped outside the container with a three-way valve, the first outlet of which leads to the tube, the second outlet leads to a waste container and the third outlet leads to a physiological saline solution reservoir. The device further includes at least one pump for pumping the physiological saline solution into the container and for pumping out the used physiological saline solution from the container after each washing cycle. 10 amniotic membranes are washed with physiological solution in this device (washing time 43 minutes in three cycles, a total of 5 liters of physiological saline solution was used). Subsequently, the amniotic membranes are removed, transferred to a pad and lyophilized at a temperature of +20 to - 65 °C and a pressure of 13.3 Pa to 60 Pa.

After lyophilization, the tissues are cut into smaller pieces according to the required size (e.g. 3x4 cm). Fatty tulle (tulle gras; polyester fabric with a layer of Vaselinum Album) of the size 6x8 cm is inserted into a manual pressing machine, a layer of lyophilized amniotic membrane is placed on top of it, and fatty tulle of the size 6x8cm with a cut corner is placed crosswise on top of it (cutting the corner serves for easier manipulation when applying to the wound). All the layers are pressed together on the manual pressing machine, which is pressed by the maximum force of the worker. Subsequently, the product is wrapped in a sterile polyethylene packaging and sterilized by radiation.

The final composition of the product containing all 5 layers:

| **Component** | **weight (g)** | |
|---|---|---|
| | 12 cm² | 1 cm² |
| First layer of polyester fabric | 0.0526 | 0.0044 |
| First layer of petroleum jelly | 0.0846 | 0.0071 |
| Non-viable amniotic tissue | 0.0199 | 0.0017 |
| Second layer of petroleum jelly | 0.0846 | 0.0071 |
| Second layer of polyester fabric | 0.0526 | 0.0044 |
| **Total** | **0.2943** | **0.0247** |

After removal of the second layer of polyester fabric, the composition of the product is as follows:

| **Component** | **weight (g)** | |
|---|---|---|
| | 12 cm² | 1 cm² |
| First layer of polyester fabric | 0.0526 | 0.0044 |
| First layer of petroleum jelly | 0.0846 | 0.0071 |
| Non-viable amniotic tissue | 0.0199 | 0.0017 |
| Second layer of petroleum jelly | 0.0677 | 0.0056 |
| **Total** | **0.2248** | **0.0188** |

### Example 2: Moisture retention

Moisture retention was determined as MVTR (moisture vapor transmission rate) according to the ČSN EN 13 726-2:2002 standard.

The test was performed according to Article 3 of the standard. Gravimetric analysis of the samples was performed before and after 24-hour and 2000-hour exposure at a temperature of 37 °C in the preparations described in the standard.

The results are shown in the following table:

| Sample | Unites | Test results¹ | Measurement uncertainty² |
|---|---|---|---|
| Product prepared according to Example 1 | g.m⁻².24 h⁻¹ | 998 | 3 |
| Fatty tulle (polyester fabric with petroleum jelly) | g.m⁻².24 h⁻¹ | 15754 | 4 |

| | | | |
|---|---|---|---|
| ¹ arithmetic average from 5 measurements; ² measurement uncertainty extended for coverage factor k=2, which corresponds to the coverage probability of ca 95 % for normal distribution. | | | |

As a comparison to the wound cover manufactured as in Example 1, fatty tulle was used, which showed an MVTR of 15.754 g/m²/day. The wound cover according to the invention showed an MVTR = 998 g/m²/day, which is 94% less than the fatty tulle.

This test proved that the wound cover according to the invention has a much higher efficiency in maintaining optimal moisture in the wound. Optimal moisture in the wound effectively supports the healing process.

### Example 3: Effect of a layer of deactivated non-viable amniotic tissue on reducing microbial contamination

Evaluation of the penetration of dry microorganisms through the product was carried out according to the EN ISO 22612:2005 standard. Within the framework of the test, samples of the wound cover prepared according to example 1 and fatty tulle (polyester fabric with petroleum jelly) were compared.

The conditions of the experiment were as follows:
- Bacterial strain: *Bacillus subtilis* - ATCC 9372
- Talc: CM3 Koltex Color (particle size 7.3 µm)
- Spore concentration: 1.0×10⁸ CFU/g talc (CFU = colony forming units)
- Vibration time: 30 minutes
- Vibration frequency: 20,800 vibrations/minute
- Number of tested samples: 10
- Number of reference samples: 2
- Agar: TGE (trypton glucose extract agar)
- Incubation period: 24 hours
- Incubation temperature: 35 °C

### Results:

Arithmetic average of colony forming units (CFU) number for 10 Petri dishes, median (M_{d}) and upper quartile (U_{q}) are shown in the following tables.

| Wound cover according to the invention (according to example 1) | | |
|---|---|---|
| Dish number | Test 1 (CFU/dish) | Test 2 (CFU/dish) |
| 1 | 7 | 68 |
| 2 | 2 | 62 |
| 3 | 0 | 1 |
| 4 | 61 | 26 |
| 5 | 7 | 4 |
| CFU Average | **28** | |
| M_{d}CFU | **16.5** | |
| U_{q}CFU | **61** | |

| Fatty tulle (comparative material) | | |
|---|---|---|
| Dish number | Test 1 (CFU/dish) | Test 2 (CFU/dish) |
| 1 | TMTC | TMTC |
| 2 | TMTC | TMTC |
| 3 | TMTC | TMTC |
| 4 | TMTC | TMTC |
| 5 | TMTC | TMTC |
| CFU Average | - | |
| M_{d}CFU | - | |
| U_{q}CFU | - | |

| | | |
|---|---|---|
| TMTC..... too many to count | | |

### Example 4: Comparison of effects of amniotic membrane and of the wound cover according to the invention

To compare the effects of the amniotic membrane alone and the wound cover according to the invention (as described in example 1), a comparison of two clinical tests was carried out.

The first clinical test was carried out with amniotic membrane alone on a group of 24 patients with diabetic foot syndrome (DFU), who were followed for 12 weeks. The median age of the wound was 24 months, the median age of the patients was 65 years, and the median area of the wounds at the beginning of the follow-up was 2.8 cm² and at the end of the follow-up 1.9 cm². The treatment success rate was 42%, with the first wound closure occurring at week 3, calculated from the initiation of treatment, and the greatest increase in the number of wound closures occurring between weeks 4 and 5. Wound pain was experienced by 12.5% of patients at the beginning of treatment, and by only 4.2% of patients at the end of treatment.

A total of 20 patients with DFU were followed for 12 weeks in the second clinical trial, carried out with the wound cover according to the invention. The median age of the wound was 12 months, the median age of the patients was 58.5 years, and the median area of the wounds at the beginning of the follow-up was 3.7 cm² and at the end of the follow-up 0.1 cm². The success rate of the treatment was 65%, when the first wound closed already in week 2 from the initiation of treatment and the greatest increase in the number of closed wounds occurred between weeks 10 and 11. Wound pain was experienced by 20.0% of patients at the beginning of treatment, and by only 15.0% of patients at the end of treatment.

In the first case, i.e. in DFUs treated with amniotic membrane alone, smaller but older wounds were observed (median age of the wound 24 months), while the age of the wound also affects the speed of wound healing. In these wounds, however, there was a faster onset of treatment effects around 6-8 weeks of treatment. This rapid onset of action is probably due to the fact that, in addition to the covering function, the amniotic membrane used for the treatment could also release proteins into the wound that lead to the acceleration of wound closure. However, the overall treatment success rate was lower (42%).

In the second case, i.e. in DFUs treated with a wound cover according to the invention, slightly larger but younger wounds were observed (median age of wounds 12 months). These wounds had a slower onset of treatment effects between weeks 10 and 11 of treatment. This delayed onset of treatment compared to the previous variant is probably due to the fact that the wound cover does not release cytokines and growth factors into the wound as is the case with amniotic membrane alone. This wound cover does not rely on this supportive function, but has a covering function and creates a natural homeostatic environment in the wound. The patient's organism then closes the wound naturally without supporting treatment. This method of treatment has a slower onset of action, but a longer-lasting effect, and an overall higher success rate of treatment (65%).

## Claims

1. Wound cover, which contains, in the following order:
- a first layer of polyester fabric,
- a first layer of petroleum jelly,
- a layer of deactivated non-viable amniotic tissue (DNAT),
- a second layer of petroleum jelly,
- a removable second layer of polyester fabric.

2. Wound cover according to claim 1, which contains, in the following order:
- the first layer of polyester fabric, in the amount of 3.7 to 9.8 mg/cm² of the wound cover,
- the first layer of petroleum j elly, in the amount of 6.5 to 14.7 mg/cm² of wound cover,
- the layer of deactivated non-viable amniotic tissue, in the amount of 0.2 to 4 mg/cm² of wound cover,
- the second layer of vaseline, in the amount of 5.5 to 14.7 mg/cm² of wound cover,
- the removable second layer of polyester fabric, in the amount of 3.7 to 9.8 mg/cm² of the wound cover.

3. Wound cover according to claim 1 or 2, wherein the polyester fabric is a woven fabric formed by polyester fibers.

4. Wound cover according to any one of claims 1 to 3, wherein the polyester fabric is formed by polyester fibers having diameters from 1 nanometer to 1000 micrometers.

5. Method of manufacturing of the wound cover according to any one of claims 1 to 4, said method comprising the following steps:
a) preparation of amniotic membranes from amnions or from placentas containing amnions,
b) washing the amniotic membranes with physiological saline solution,
c) optional washing of amniotic membranes in an antibiotic solution,
d) lyophilization of the amniotic membrane, preferably at a temperature from +20 to - 65 °C and a pressure from 13.3 Pa to 60 Pa,
e) fragmentation of the lyophilized amniotic membrane into the required dimensions,
f) assembling the lyophilized amniotic membrane with layers of petroleum jelly and layers of polyester fabric, preferably at a temperature of 15 to 25 °C and a pressure of 101 kPa to 10 MPa, compressing the assembled layers, and placing them in sterile packaging,
g) final sterilization of the wound cover by gamma radiation with a dose of 14 to 20 kGy, preferably at a temperature from 0 to 35 °C.

6. The method according to claim 5, wherein the step of washing the amniotic membranes with physiological saline solution and/or the optional step of washing the amniotic membranes in an antibiotic solution is carried out by means of a washing device comprising a container with a lid, the container containing a concentrically placed perforated basket connected to a vertical central axis passing through the lid and provided with a driving means located in the lid, and the device is further provided with a fluid supply and discharge tube which opens at the bottom of the container, wherein the said tube is provided with a three-way valve outside the container, the first outlet of the three-way valve leads to the tube, the second outlet leads to a waste container, and the third outlet leads to a physiological saline reservoir, and wherein the device further contains at least one pump for pumping the physiological saline solution into the container and for pumping out the used physiological saline solution from the container after each washing cycle.
